# EUROPEAN PATENT SPECIFICATION

(11) **EP 2 308 369 B1**
(45) Date of publication and mention of the grant of the patent: **03.06.2015**
(21) Application number: 10251782.8
(22) Date of filing: 12.10.2010
(51) Int. Cl.: A61B 3/16, A61B 5/022

(54) **Method of estimating ocular perfusion pressure**
Verfahren zur Bestimmung des okulären Perfusionsdrucks
Procédé pour estimer la pression de perfusion oculaire

(30) Priority: 12.10.2009 US 577470
(43) Date of publication of application: 13.04.2011
(73) Proprietor: Falck Medical, Inc., Mystic, Connecticut 06355 (US)
(72) Inventor: Falck, Francis Y., Jr., Stonington, Connecticut 06378 (US); Falck, Robert W., Pawcatuck, Connecticut 06379 (US)
(74) Representative: Jones, Graham Henry

(56) References cited:
- GB-A- 728 775
- US-A- 3 903 871
- US-A- 3 929 124
- US-A- 4 281 662
- GOLDMAN R M ET AL: "THE EFFECTS OF OSCILLATING INVERSION ON SYSTEMIC BLOOD PRESSURE, PULSE, INTRAOCULAR PRESSURE, AND CENTRAL RETINAL ARTERIAL PRESSURE", PHYSICIAN AND SPORTSMEDICINE, MCGRAW-HILL, MINNEAPOLIS, US, vol. 13, no. 3, 1 March 1985 (1985-03-01), pages 93-96, XP008056194, ISSN: 0091-3847

## Description

### BACKGROUND

No available instruments can directly measure the pressure of the central retinal artery of an eye and thereby estimate ocular perfusion pressure. If it were available, an accurate estimate of ocular perfusion pressure could give a clinician important information on a rate of flow of blood into the eye. This could be valuable in assessing the health of an eye, assessing the health of blood vessels supplying the eye, and help in devising treatments for eye diseases. For lack of anything better, the usual procedure is to estimate central retinal artery pressures from measures of brachial blood pressure. For example, a normal mean upright brachial blood pressure of 100mm Hg can be multiplied by 60% estimate a 60mm Hg blood presssure in a central retinal artery of an ipsilateral eye. Subtracting a normal mean intraocular pressure (MIOP) of 16mm Hg then yields a perfusion pressure of 44mm Hg. This estimate assumes no abnormality in the arteries distal to the brachial artery. Since this assumption can commonly be wrong, this method of estimating is not reliable. US 4 281 662 discloses a method of estimating retinal artery pressure by using a microprocessor controlled tonometer, the method comprising using the tonometer to determine a mean intraocular pressure including a diastolic value and a systolic value having an ocular pulse amplitude.

### SUMMARY

We have found a simple and reasonably accurate way of estimating ocular perfusion pressure by using a microprocessor-controlled tonometer that can measure diastolic IOP (DIOP), systolic IOP (SIOP), and ocular pulse amplitude (OPA) as the difference between diastolic and systolic IOP. Preferred embodiments and operating methods for such a tonometer are suggested in the following U.S. Patents and applications: 6,179,779, Replacement Prism System for Applanation Tonometer, 6,471,647, Method of Operating a Tonometer; 6,736,778, Replacement System for Applanation Tonometer, 7,153,267, Ophthalmologic Applanation Prism Replacement System; 7,473,231, Method and Apparatus for Examining an Eye; and 7,479,109, Ophthalmologic Applanation Cornea Contactor Replacement System for Eye Examining Instrument; and U.S. Publication 2009-0103047, Tonometer Using Camera and Ambient Light.

A tonometer such as proposed in these references can be operated to estimate ocular perfusion pressure directly from an eye. The tonometer can produce a more realistic estimate of mean central retinal artery pressure (MCRAP) by using at least two different alternatives. A tonometer operated correctly can also be programmed to distinguish between measurements falling within a normal range for a healthy eye and measurements falling outside the normal range and thus requiring a re-measure or possibly some remedial action. A tonometer used for estimating ocular perfusion pressure preferably includes a microprocessor that can control operations and manage the data generated, can make whatever calculations are necessary, and can use look-up tables and make data comparisons to produce outputs usable to an operator. The goals of the inventive way of estimating ocular perfusion pressure include speed and convenience using an available tonometer, low cost and reliability, and providing substantially improved information to a clinician examining the health of the eye and the vascular system supplying it

### DRAWINGS

Fig.1 is a schematic diagram of method steps leading to an ocular perfusion pressure estimate, by using one preferred alternative.
Fig.2 is a schematic diagram of method steps leading to an ocular perfusion pressure estimate, using an alternative preferred embodiment.
Fig.3 schematically illustrates a tonometer and the tonometer functions necessary to practice the inventive method.
Fig.4 is a graph of a signal generated by the tonometers of FIGS. 1- 3 in practicing the inventive method.

### DETAILED DESCRIPTION

Estimating ocular perfusion pressure requires estimating blood pressure in a central retinal artery of an eye being examined. A mean pressure for the central retinal artery (MCRAP) can be reached by at least two preferred methods and by variations on these methods as explained below. A reliable estimate of MCRAP and a measure of mean intraocular pressure (MIOP) can then produce an estimate of ocular perfusion pressure simply by subtracting M IOP from MCRAP.

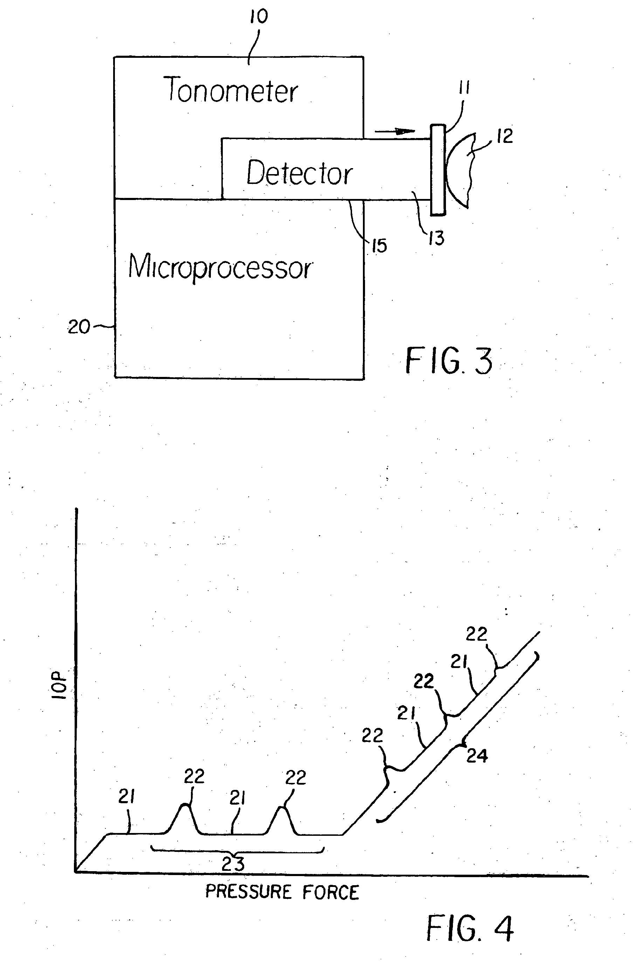

Each estimating method begins with tonometer 10 pressing a surface 11 lightly against a cornea 12 of an eye with a variable pressing force produced by element 13 and indicated by the arrow in FIG.3. While this happens, pulses occur in a central retinal artery of an eye. A detector 15 receives reflected light as a function of a size of an area of cornea 12 deformed by surface 11 as the central retinal artery pulses occur. Detector 15 converts the received light signals into electric signals transmitted to microprocessor 20, which is programmed to estimate MCRAP and ocular perfusion pressure from the available signals. Preferred embodiments for tonometer 10 are listed in the Summary above.

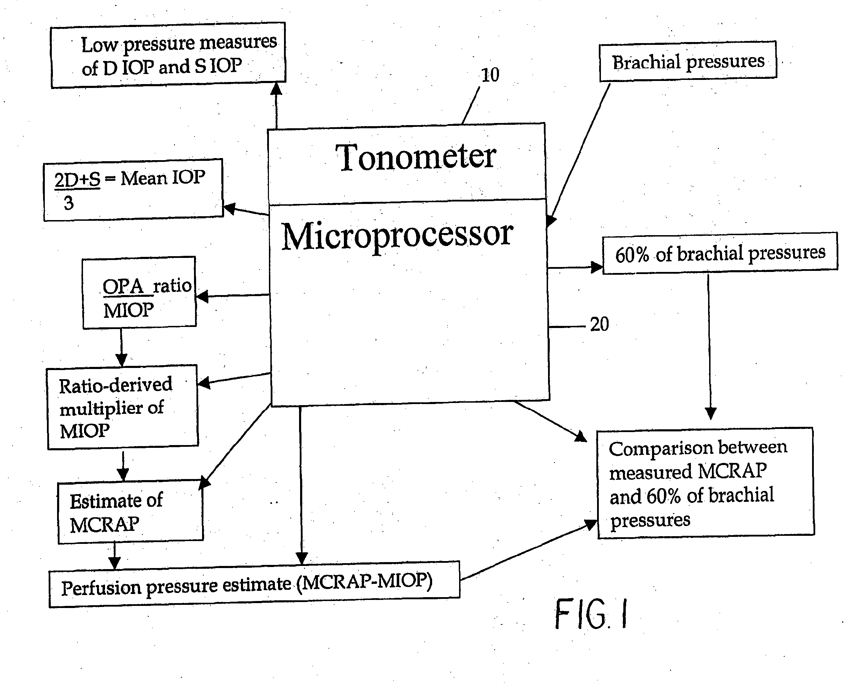

One preferred way for tonometer 10 to operate is schematically set out in FIG.1. It begins with low pressure measures of intraocular pressure (IOP) made while surface 11 is pressed lightly against cornea 12. The pressure of surface 11 against cornea 12 is preferably small enough forthis purpose not to increase IOP values within the eye being examined. The IOP measures include diastolic values and systolic values 22 (FIG.4), which appear each time a pulse occurs in a central retinal artery ofan eye being examined. Tonometer 10 calculates an MIOP from signal region 23 generated during low pressure force of surface 11 against cornea 12. The MIOP value is preferably made by multiplying the diastolic value 21 by 2, adding the systolic value 22, and dividing by 3. An MIOP value for a normal healthy eye is about 16mm Hg. In low pressure signal range 23, where IOP of the eye is not increased, an ocular pulse amplitude (OPA) of systolic pulses 22 has been commonly recognized as about 2mm Hg for a healthy eye. Data collected empirically from our examination of many eyes using the preferred tonometers and methods mentioned above has put OPA somewhat higher than 2mm Hg in the range of 2-3mm Hg.

From these measured values, microprocessor 20 can calculate an estimate of MCRAP. A preferred way of doing this is to form a ratio of OPA divided by MIOP. This ratio is preferably multiplied by 100 to become expressed as a percentage. The ratio X can be written X = OPA/MIOP x 100. For a normal eye having an MIOP of 16mm Hg and an OPA of 2.5mm Hg, the ratio X =15.625%.

The ratio X can be used to derive a multiplier Y, which is based on empirical study of many normal eyes supplied by normal vascular systems. The multiplier is also chosen so that normal eyes fall within the healthy range of 52mm Hg to 63mm Hg for MCRAP. The multiplier Y derived from the ratio X can be expressed as Y = CX, with C varying from about 0.2 to about 0.3. The Y multiplier thus reduces the X ratio to about one-fourth to one-fifth, depending partly on whether the calculation aims at the middle of the healthy range for MCRAP or at the minimum healthy range of MCRAP.

The microprocessor then multiplies MIOP by the multiplier to produce the estimate of MCRAP. lf this estimate falls below 52mm Hg, it indicates a potential problem. The first action is preferably to re-measure and recalculate. If this produces another low estimate, then further investigation of the vascular supply to the eye may be warranted.

As the ratio X deviates from normal conditions of a mean IOP of 16mm Hg and an OPA of 2mm Hg, then the Y multiplier also changes. The Y multiplier is also applied to whatever MIOP is actually measured, to yield an MCRAP estimate based on the conditions measured for any specific eye. MCRAP estimates smaller than 52mm Hg are of special interest because they may signify vascular disease or abnormality in the supply of blood to an eye. This can lead to further measurements and other investigations.

The MCRAP estimate reached by the method described above and illustrated in FIG.1 can be corroborated by an MCRAP estimate based on brachial blood pressures. These can be measured for each arm of a person and preferably entered into microprocessor 20, which then calculates 60% of the brachial pressures of each arm. The microprocessor can then compare the measured MCRAP estimate with the brachial blood pressure estimate af MCRAP to see whether discrepancies exist Most of these discrepancies will involve the calculated MCRAP being lower than 60% of the ipsilateral brachial blood pressure, which is often worth investigating further. Discrepancies can exist, however, without indicating poor health. For example, a person who is physically fit with a blood pressure significantly lower than normal could have a low calculated MCRAP that is explained by low brachial blood pressure.

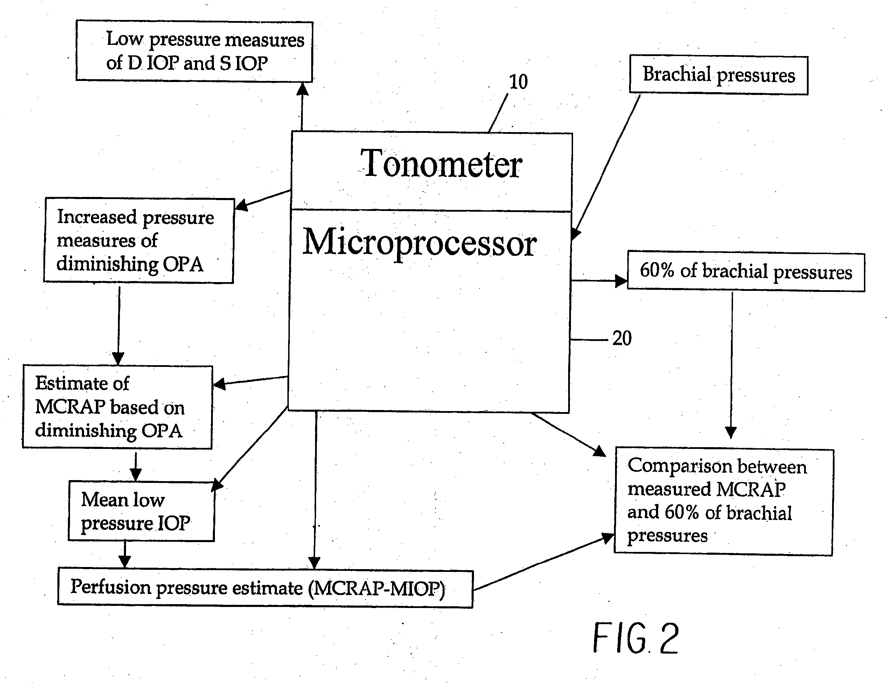

An alternative preferred method illustrated in FIG.2 involves a calculated MCRAP estimate based on different measurements of the eye, with corroborating brachial pressures being used for a comparison, as explained above.

After the diastolic IOP, systolic IOP, and OPA are measured by tonometer 10 as explained above, then new measurements are made during increased pressure on the eye. Tonometer 10 presses surface 11 more forcefully against cornea 12 to raise the IOP within the eye. The increasing IOP is represented by the inclined portion 24 of the signal shown in FIG.4. Microprocessor 20 collects from detector 15 information on ocular pulse amplitude (OPA), which is the height of a systolic pulse 22 above a diastolic base line 21. As pressure of surface 11 against cornea 12 increases, the IOP rises as shown in signal region 24, while microprocessor 20 gathers information on a corresponding reduction in OPA of systolic pulses 22.

Experience has shown that systolic pulses 22 diminish in OPA as IOP increases in signal region 24. Our many observations have also shown that the relationship between increasing IOP and diminishing OPA, in the regions of interest for eye examination, is linear and inverse. This fact, together with information on expected upper and lower ranges, is used by microprocessor 20 to calculate an initial estimate of mean central retinal artery pressure (MCRAP). For a normal healthy eye and normal blood pressure, the estimate of MCRAP is reached, and microprocessor 20 calculates ocular perfusion pressure by subtracting mean IOP from MCRAP. Measurements of MIOP and OPA that depart from normal can lead to different MCRAP estimates and to different perfusion pressure estimates that can be investigated further.

As shown in signal region 24 of FIG.4, systolic pulses 22 are expected to diminish in amplitude as IOP increases. The reduction in OPA as IOP increases is expected to be linear and inversely proportional to increases in IOP. Data collected in signal region 24 can then be compared with these expectations to see whether blood flow is normally available to the eye being examined. Departures from the expectations can indicate blood flow problems, which mostly involve less blood flow than expected and desired A regression analysis can produce look-up tables that microprocessor 20 can use to adjust the initial estimate of MCRAP based on the signals received from region 24. For example, OPA could appear normal in signal region 23, and could reduce faster than the expected inverse linear relationship in signal region 24. This would suggest a constricted blood flow to the eye that would warrant clinical intervention.

The right side of the diagram of FIG.1 suggests a preferred way of corroborating a calculated MCRAP estimate. This involves taking brachial blood pressures in an upright position for each arm of a patient. These measures include both diastolic and systolic values, which are preferably entered into microprocessor 20 oftonometer 10. The microprocessor can then calculate mean brachial blood pressure values by multiplying diastolic values by 2, adding systolic values, and dividing by 3. Alternatively, the brachial blood pressure readings can be converted to mean brachial pressures before entry into microprocessor 20.

From our many previous measurements, and because of the approximately 25cm difference in height of the eye above the heart for an upright person, we have found that MCRAP for an eye should be about 60% of ipsilateral brachial blood pressure. Microprocessor 20 can then calculate an estimate of M CRAP based on 60 % of a mean ipsilateral brachial blood pressure, and microprocessor 20 can compare the calculated estimate of MCRAP based on OPA/IOP data with the estimate of MCRAP based on 60% of mean brachial blood pressures. To be clear, there is a set of brachial blood pressures for each arm and a set of IOP pressures for each eye and the comparison is made between ipsilateral measures for the right arm and the right eye and measures for the left arm and the left eye.

If comparison of the MCRAP value derived from IOP measures differs significantly from the MCRAP estimates based on brachial blood pressures, then microprocessor 20 can point this out to an operator by a signal of some sort. This can lead to re-measures and re-estimates or possibly other diagnostic investigations to account for the discrepancy.

As illustrative of the estimating method, consider a normal healthy eye with a mean IOP of 16mm Hg. With a normal healthy blood pressure, such an eye would have an OPA of about 2.5mm Hg This can afford a ratio of a mean OPA of 2mm Hg divided bya mean IOP of 16mm Hg to produce 0.156, which can be multiplied by 100 to reach 15.6%. With such a normal eye and blood pressure, we have found that MCRAP should range from 52 to 61mm Hg. This can be based on 0.6 times the normal healthy range for brachial blood pressure. We then derive a Y multiplier from the X value 15.6% using a constant in the 0.2 to 0.3 range, for example 0.21. We apply this multiplier to the X percentage 15.6 to produce a multiplier of 3.276. We then multiply MIOP of 16 by the derived multiplier of 3.276 to estimate MCRAP at 52.4, which is just within the low end of the healthy range for MCRAP. Using the lower end of the normal range (52mm Hg) is convenient for this purpose, because we are looking for possible vascular impairment that might reduce blood How below the normal range. For an eye having normal healthy values, microprocessor 20 can calculate the measured OPA divided by the measured IOP and apply the derived multiplier to see whether the eye meets the normal minimum of 52mm Hg MCRAP. Subtracting a mean IOP value from MCRAP then gives a realistic, eye-measurement-based estimate of perfusion pressure. For a normal eye having a mean IOP of 16, and an estimated MCRAP of 52, the estimated perfusion pressure is 36. Estimates lower than this can indicate vascular disease warranting further investigation.

A microprocessor making the calculations described above can then notify the operator of the tonometer if the estimated MCRAP value or perfusion pressure value falls below the normal range. This can lead to a re-measurement or some other investigation to identify the discrepancy.

As mean OPA values decrease with increasing mean IOP in region 24 of the signal of FIG. 4, the multiplier of the ratio between OPA over IOP also changes, based primarily on an inverse linear relationship between OPA and IOP. The value picked for the changing multiplier is preferably based on a look-up table, which can be prepared based on regression analysis of many measurements.

## Claims

1. A method of estimating mean central retinal artery pressure (MCRAP) by using a microprocessor controlled tonometer (10), the method comprising:
using the tonometer (10)by pressing a surface (11) against a cornea to determine a mean intraocular pressure (MIOP) including a diastolic value and a systolic value having an ocular pulse amplitude (OPA)
using the microprocessor (20) to calculate a ratio between OPA and MIOP;
using the microprocessor (20) to derive a multiplier from the ratio; and
using the microprocessor (20) to multiply MIOP by the derived multiplier to produce an estimate of mean central retinal artery pressure (MCRAP).

2. A method according to claim 1 including programming the tonometer (10) to calculate MIOP based on 2 times the diastolic IOP plus the systolic IOP divided by 3.

3. A method according to claim 1 or claim 2 including using the microprocessor (20) to estimate ocular perfusion pressure by subtracting MIOP from MCRAP.

4. A method of estimating ocular perfusion pressure of any one of claims 1-3 using light pressure of a corneal engaging surface (11) against the cornea of the eye (12) being examined while determining MIOP by:
pressing the corneal engaging surface (11) against the cornea of the eye (12) with increased force while measuring diminishment of an amplitude of OPA; and
using the microprocessor (20) to estimate MCRAP from an approximately linear and inverse relationship between increasing cornea engaging surface pressure and diminishing OPA.

5. A method according to any one of claims 1-3 including estimating MCRAP at 60% of an ipsilateral brachial blood pressure of person in an upright position, and comparing the MCRAP estimate based on brachial pressure with the measured MCRAP estimate.

6. A method according to claim 5 including entering the brachial blood pressures into the microprocessor (20), and programming the microprocessor to make the MCRAP estimate comparison.

7. A method according to claim 6 including arranging the microprocessor (20) to display the estimate of MCRAP in millimeters of Hg.

8. A tonometer (10) having a cornea engaging surface (11) and a microprocessor (20) programmed to perform the method steps according to any one of claims 1-7.

## Patentansprüche

1. Verfahren zum Abschätzen des mittleren zentralen Retinalarteriendrucks (MCRAP) unter Verwendung eines mikroprozessorgesteuerten Tonometers (10), wobei das Verfahren umfasst:
Verwenden des Tonometers (10) durch Drücken einer Oberfläche (11) gegen eine Hornhaut, um den mittleren Augeninnendruck (MIPO) zu bestimmen, einschließlich eines diastolischen Werts und eines systolischen Werts mit einer okularen Pulsamplitude (OPA);
Verwenden des Mikroprozessors (20) zum Berechnen des Verhältnisses zwischen OPA und MIOP;
Verwenden des Mikroprozessors (20) zum Ableiten eines Multiplikators aus dem Verhältnis; und
Verwenden des Mikroprozessors (20) zum Multiplizieren des MIOP mit dem abgeleiteten Multiplikator, um eine Abschätzung des mittleren zentralen Retinalarteriendrucks (MCRAP) zu erhalten.

2. Verfahren gemäß Anspruch 1, umfassend Programmieren des Tonometers (10) zum Berechnen des MIOP auf der Grundlage von 2-mal dem diastolischen IOP plus dem systolischen IOP geteilt durch 3.

3. Verfahren gemäß Anspruch 1 oder Anspruch 2, umfassend Verwenden des Mikroprozessors (20) zum Abschätzen des okularen Perfusionsdrucks durch Subtrahieren des MIOP von dem MCRAP.

4. Verfahren zum Abschätzen des okularen Perfusionsdrucks gemäß einem der Ansprüche 1-3 unter Verwendung von leichtem Druck einer Hornhaut-berührenden Oberfläche (11) gegen die Hornhaut des untersuchten Auges (12), während der MIOP bestimmt wird durch:
Drücken der Hornhaut-berührenden Oberfläche (11) gegen die Hornhaut des Auges (12) mit zunehmender Kraft, während die Verringerung der Amplitude der OPA gemessen wird; und
Verwenden des Mikroprozessors (20) zum Abschätzen des MCRAP aus einer näherungsweise linearen und inversen Beziehung zwischen zunehmendem Druck der Hornhaut-berührenden Oberfläche und Verringerung der OPA.

5. Verfahren gemäß einem der Ansprüche 1-3, einschließlich Abschätzen des MCRAP als 60 % des ipsilateralen brachialen Blutdrucks einer Person in aufrechter Position und Vergleichen der MCRAP-Abschätzung auf der Grundlage des brachialen Drucks mit der gemessenen MCRAP-Abschätzung.

6. Verfahren gemäß Anspruch 5, einschließlich Eingeben des brachialen Blutdrucks in den Mikroprozessor (20) und Programmieren des Mikroprozessors, um den Vergleich der MCRAP-Abschätzungen durchzuführen.

7. Verfahren gemäß Anspruch 6, einschließlich Einrichten des Mikroprozessors (20) zum Anzeigen der Abschätzung des MCRAP in Millimeter Hg.

8. Tonometer (10) mit einer Hornhaut-berührenden Oberfläche (11) und einem Mikroprozessor (20), der zum Durchführen der Verfahrensschritte gemäß einem der Ansprüche 1-7 programmiert ist.

## Revendications

1. Procédé d'estimation de la pression moyenne de l'artère rétinienne centrale (MCRAP) au moyen d'un tonomètre commandé par microprocesseur (10), le procédé comprenant les étapes suivantes :
utiliser le tonomètre (10) en pressant une surface (11) contre une cornée pour déterminer une pression intraoculaire moyenne (MIOP) comprenant une valeur diastolique et une valeur systolique ayant une amplitude d'impulsion oculaire (OPA),
utiliser le microprocesseur (20) pour calculer un rapport entre l'OPA et la pression MIOP ;
utiliser le microprocesseur (20) pour dériver un multiplicateur à partir du rapport ; et
utiliser le microprocesseur (20) pour multiplier la pression MIOP par le multiplicateur dérivé pour produire une estimation de la pression moyenne de l'artère rétinienne centrale (MCRAP).

2. Procédé selon la revendication 1, comprenant de programmer le tonomètre (10) pour calculer la pression MIOP sur la base de deux fois la pression IOP diastolique plus la pression IOP systolique divisée par trois.

3. Procédé selon la revendication 1 ou la revendication 2, comprenant d'utiliser le microprocesseur (20) pour estimer la pression de perfusion oculaire en soustrayant la pression MIOP de la pression MCRAP.

4. Procédé d'estimation de la pression de perfusion oculaire selon l'une quelconque des revendications 1 à 3 utilisant une légère pression d'une surface d'engagement de cornée (11) contre la cornée de l'oeil (12) en cours d'examen lors de la détermination de la pression MIOP :
en pressant la surface d'engagement de la cornée (11) contre la cornée de l'oeil (12) avec une force accrue, tout en mesurant une diminution de l'amplitude de l'OPA ; et
en utilisant le microprocesseur (20) pour estimer la pression MCRAP à partir d'une relation approximativement linéaire et inverse entre l'augmentation de la pression de la surface d'engagement de la cornée et la diminution de l'OPA.

5. Procédé selon l'une quelconque des revendications 1 à 3, comprenant d'estimer la pression MCRAP à 60 % d'une pression sanguine brachiale ipsilatérale d'une personne en position verticale, et de comparer l'estimation de la pression MCRAP sur la base de la pression brachiale avec l'estimation de la pression MCRAP mesurée.

6. Procédé selon la revendication 5, comprenant d'entrer les pressions sanguines brachiales dans le microprocesseur (20), et de programmer le microprocesseur pour effectuer la comparaison d'estimation de la pression MCRAP.

7. Procédé selon la revendication 6, comprenant d'arranger le microprocesseur (20) pour afficher l'estimation de la pression MCRAP en millimètres de mercure.

8. Tonomètre (10) comprenant une surface d'engagement de cornée (11) et un microprocesseur (20) programmé pour exécuter les étapes de procédé selon l'une quelconque des revendications 1 à 7.
